# EUROPEAN PATENT APPLICATION

(11) **EP 4 768 076 A1**
(43) Date of publication of application: **01.07.2026**
(21) Application number: 25227895.7
(22) Date of filing: 31.12.2025
(51) Int. Cl.: A61N 5/10

(54) **DETERMINATION METHOD FOR ADAPTIVE RADIOTHERAPY AND TRAINING METHOD FOR TRIGGER ANALYSIS MODEL**

(30) Priority: 31.12.2024 CN 202412000014
(71) Applicant: Shanghai United Imaging Healthcare Co., Ltd., Shanghai 201807 (CN)
(72) Inventor: CHENG, Songyang, Shanghai, 201807 (CN); CAO, Ran, Shanghai, 201807 (CN); ZHOU, Jingjie, Shanghai, 201807 (CN)
(74) Representative: Murgitroyd & Company

(57) **Abstract**

Disclosed is a determination method for adaptive radiotherapy. The method includes: obtaining a current fraction image corresponding to a current radiotherapy fraction of a target subject, and obtaining an initial image configured for determining an initial radiotherapy plan for the target subject; determining a trigger analysis result according to the current fraction image, the initial image, and a trigger analysis model; and determining whether to perform adaptive radiotherapy based on the trigger analysis result.

## Description

### TECHNICAL FIELD

The present disclosure relates to the field of radiotherapy technology, and in particular, to a determination method for adaptive radiotherapy, a training method for a trigger analysis model, and a storage medium.

### BACKGROUND

With the development of radiotherapy technology, Image-Guided Radiotherapy (IGRT) and Adaptive Radiotherapy (ART) have become two important methods in radiotherapy. An adaptive radiotherapy plan (referred to as an adaptive plan) is a radiotherapy method that involves re-adjusting the plan during the patient's radiotherapy course, based on changes in position, size, or treatment progression, of a tumor, which is a relatively complex and time-consuming process.

Conventionally, determination methods for triggering an adaptive plan involve a user analyzing IGRT images on-site during treatment implementation based on clinical experience to determine whether to trigger an adaptive plan.

However, the conventional determination methods for triggering an adaptive plan have the technical problem of low efficiency.

### SUMMARY

Accordingly, it is necessary to address the above technical problems and provide a determination method for adaptive radiotherapy, a training method for a trigger analysis model, an apparatus, a computer device, a computer-readable storage medium, and a computer program product, improving the judgment efficiency and rationality of adaptive plan triggering.

In a first aspect, a determination method for adaptive radiotherapy is provided. The determination method for adaptive radiotherapy includes:
obtaining a current fraction image corresponding to a current radiotherapy fraction of a target subject, and obtaining an initial image configured for determining a previous radiotherapy plan for the target subject;
determining a trigger analysis result according to the current fraction image, the initial image, and a trigger analysis model; and
determining whether to perform adaptive radiotherapy based on the trigger analysis result.

In an embodiment, the trigger analysis result includes at least one of trigger attention parameters or adaptive radiotherapy trigger determination information, and the trigger attention parameter includes benefit information of one or more dose indicators.

In an embodiment, the trigger analysis model includes a first trigger analysis model. Determining the trigger analysis result according to the current fraction image, the initial image, and a trigger analysis model includes:
performing image registration on the current fraction image and the initial image to obtain a registered image;
delineating a region of interest in the registered image to obtain a delineated image, where the delineated image includes a delineation result of the region of interest; and
inputting the delineated image into the first trigger analysis model to output the trigger analysis result.

In an embodiment, the first trigger analysis model includes a first feature extraction network and a trigger analysis network. Inputting the delineated image into the first trigger analysis model to output the trigger analysis result includes:
inputting the delineated image and the initial image into the first feature extraction network for feature extraction to obtain target feature information; and
inputting the target feature information into the trigger analysis network to output the trigger analysis result, where the initial image includes an original delineation result of the region of interest.

In an embodiment, the target feature information includes at least one of morphological difference feature information, image difference feature information, or registration difference feature information, based on the region of interest.

In an embodiment, the trigger analysis model includes a second trigger analysis model. Determining the trigger analysis result according to the current fraction image, the initial image, and a trigger analysis model includes:
performing a first delineation on the region of interest in the current fraction image according to an original delineation result in the initial image to obtain a first delineated image;
performing a second delineation on the region of interest in the current fraction image to obtain a second delineated image; and
inputting the first delineated image and the second delineated image into the second trigger analysis model to output the trigger analysis result.

In an embodiment, performing the first delineation on the region of interest in the current fraction image according to the original delineation result in the initial image to obtain the first delineated image includes performing a rigid copy on the original delineation result from the initial image to the current fraction image to obtain the first delineated image containing the original delineation result.

In an embodiment, the second trigger analysis model includes a second feature extraction network and a decision network. Inputting the first delineated image and the second delineated image into the second trigger analysis model to output the trigger analysis result includes:
inputting the first delineated image into the second feature extraction network for feature extraction to obtain first feature information;
inputting the second delineated image into the second feature extraction network for feature extraction to obtain second feature information; and
inputting the first feature information and the second feature information into the decision network to output the trigger analysis result.

In an embodiment, the trigger analysis result includes a trigger attention parameter. The method further includes:
performing a weighted summation on the benefit information of each dose indicator in the trigger attention parameters to obtain comprehensive benefit information; and
determining adaptive radiotherapy trigger determination information according to the comprehensive benefit information and a preset benefit information threshold.

In a second aspect, a training method for a trigger analysis model is provided. The trigger analysis model is applied to determination for the adaptive radiotherapy. The training method includes:
obtaining training sample data, where the training sample data includes initial sample images used when determining an initial radiotherapy plan for different sample subjects, fraction sample images of the sample subjects at different radiotherapy fractions, and sample trigger analysis results corresponding to the different radiotherapy fractions; and
training an initial trigger analysis network based on the training sample data to obtain the trigger analysis model, where the trigger analysis model is configured to output a trigger analysis result for determining whether to perform the adaptive radiotherapy.

In an embodiment, the sample trigger analysis result includes sample trigger attention parameters, and the sample trigger attention parameters include benefit information labels for one or more dose indicators. The training method for the trigger analysis model further includes:
for each fraction sample image, obtaining indicator differences of each dose indicator obtained after performing an image-guided radiotherapy plan and an adaptive plan at the corresponding radiotherapy fraction of the fraction sample image, respectively; and
performing normalization on the indicator differences of the respective dose indicators to obtain benefit information labels for one or more dose indicators corresponding to the fraction sample image.

In a third aspect, a determination apparatus for adaptive radiotherapy is provided. The apparatus includes:
an obtaining module, configured to obtain a current fraction image corresponding to a current radiotherapy fraction of a target subject, and obtain an initial image used when determining a previous radiotherapy plan for the target subject;
a first determining module, configured to determine a trigger analysis result according to the current fraction image, the initial image, and a trigger analysis model; and
a second determining module, configured to determine whether to execute adaptive radiotherapy based on the trigger analysis result.

In a fourth aspect, a training apparatus for a trigger analysis model is provided. The trigger analysis model is applied to determination for the adaptive radiotherapy. The training apparatus for the trigger analysis model includes:
a first obtaining module, configured to obtain training sample data, where the training sample data includes initial sample images used when determining an initial radiotherapy plan for different sample subjects, fraction sample images of the sample subjects at different radiotherapy fractions, and sample trigger analysis results corresponding to the different radiotherapy fractions; and
a training module, configured to train an initial trigger analysis network based on the training sample data to obtain the trigger analysis model, where the trigger analysis model is configured to output the trigger analysis result for determining whether to perform the adaptive radiotherapy.

In a fifth aspect, a computer device is provided. The computer device includes a memory and a processor, where the memory stores a computer program. The processor, when executing the computer program, implements the determination method for adaptive radiotherapy according to the first aspect or the training method for the trigger analysis model according to the second aspect.

In a sixth aspect, a non-transitory computer-readable storage medium is provided. The non-transitory computer-readable storage medium has a computer program stored thereon. The computer program, when executed by a processor, implements the determination method for adaptive radiotherapy according to the first aspect or the training method for the trigger analysis model according to the second aspect.

In a seventh aspect, a computer program product is provided. The computer program product includes a computer program. The computer program, when executed by a processor, implements the determination method for adaptive radiotherapy according to the first aspect or the training method for the trigger analysis model according to the second aspect.

### BRIEF DESCRIPTION OF DRAWINGS

FIG. 1 is a diagram of an application environment of a determination method for adaptive radiotherapy in an embodiment.
FIG. 2 is a schematic flowchart of a determination method for adaptive radiotherapy in an embodiment.
FIG. 3 is a schematic flowchart of a determination method for adaptive radiotherapy in another embodiment.
FIG. 4 is a schematic flowchart of a determination method for adaptive radiotherapy in another embodiment.
FIG. 5 is a schematic flowchart of a determination method for adaptive radiotherapy in yet another embodiment.
FIG. 6 is a schematic flowchart of a determination method for adaptive radiotherapy in still another embodiment.
FIG. 7 is a schematic flowchart of a training method for a trigger analysis model in an embodiment.
FIG. 8A is a schematic first application diagram of a first trigger analysis model in an embodiment.
FIG. 8B is a schematic second application diagram of a first trigger analysis model in an embodiment.
FIG. 9A is a schematic application diagram of a second trigger analysis model in an embodiment.
FIG. 9B is a schematic training diagram of a second trigger analysis model in an embodiment.
FIG. 10 is a structural block diagram of a determination apparatus for adaptive radiotherapy in an embodiment.
FIG. 11 is a structural block diagram of a training apparatus for a trigger analysis model in an embodiment.
FIG. 12 is an internal structural diagram of a computer device in an embodiment.

### DETAILED DESCRIPTION

To make the objectives, technical solutions, and advantages of the present disclosure clear, the following further describes the present disclosure in detail with reference to the accompanying drawings and embodiments. It should be understood that, the specific embodiments described herein are merely used to explain, but are not intended to limit, the present disclosure.

With the development of radiotherapy technology, Image-Guided Radiotherapy (IGRT) and Adaptive Radiotherapy (ART) have become two important methods in radiotherapy. An adaptive radiotherapy plan (referred to as an adaptive plan) is a radiotherapy plan that involves re-adjusting the plan during the patient's radiotherapy course based on changes in position, size, or treatment progression, of a tumor. The main advantage of the adaptive plan is effectively alleviating the problem of missing targets caused by tumor shrinkage, tumor movement, etc., during the treatment, thereby reducing the radiation dose to normal tissues surrounding the tumor, effectively reducing side effects in organs at risk, and thus improving patient treatment outcomes.

However, the process of performing radiotherapy using adaptive plans is relatively complex and time-consuming. During the implementation of adaptive radiotherapy, users, physiotherapists, and technicians need to participate together and cooperate with each other to re-delineate target volumes, optimize plan evaluation, and calculate doses based on patient data in as short a time as possible, which places high demands on clinical resources. Compared with conventional image-guided radiotherapy, the radiotherapy using an adaptive plan consumes more medical resources. In addition, during the treatment, due to factors such as an insignificant tumor shrinkage and a subtle displacement of a target volume, in some treatment fractions, the benefits of implementing the adaptive radiotherapy are minimal, resulting in unnecessary waste of medical resources. Therefore, how to maximize patient benefits while reasonably utilizing clinical resources has become an important problem.

Conventional determination method for triggering an adaptive plan often requires scanning medical images of the patient on-site during treatment implementation, and then a user determines whether to implement an adaptive radiotherapy plan for the patient on-site based on the registered images and clinical experience. Since the user needs to be on-site during the treatment, the conventional determination method for triggering the adaptive plan has a problem of low efficiency. In addition, different users may have subjective differences in their determination results for triggering adaptive plans, which may also lead to poor consistency in trigger determination.

Accordingly, a determination method for adaptive radiotherapy is provided in an embodiment of the present disclosure. The determination method for adaptive radiotherapy introduces machine learning algorithms and, based on a changes in a target volume of a patient or an organ at risk of the patient during respective radiotherapy fractions, automatically determines whether to trigger online adaptive radiotherapy and automatically obtains trigger attention parameters used for adaptive trigger determination, providing users with more specific reference information for determining whether an adjustment for the adaptive plan is required, thereby improving the rationality and interpretability of the trigger determination. This method can shorten the trigger determination time for an online adaptive plan and address the problem of poor consistency in the trigger determination.

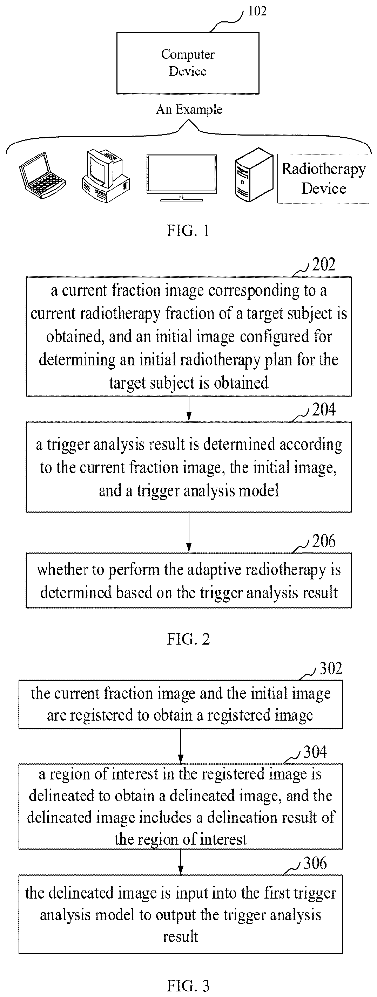

In an embodiment, the determination method for adaptive radiotherapy can be applied in an application environment as shown in FIG. 1. A computer device 102 may be a terminal, a server, or a radiotherapy device. The terminal may be, but is not limited to, a personal computer, a laptop computer, a smartphone, a tablet computer, or a portable wearable device. The wearable device includes a smart watch, a smart band, a head-mounted device, etc. The server can be implemented as an independent server or a server cluster composed of multiple servers.

In an embodiment, as shown in FIG. 2, the determination method for adaptive radiotherapy includes the following Step 202 to Step 206.

In Step 202, a current fraction image corresponding to a current radiotherapy fraction of a target subject is obtained, and an initial image used when determining an initial radiotherapy plan for the target subject is obtained.

In an embodiment, the target subject refers to a patient targeted when the determination method for adaptive radiotherapy is performed.

In an embodiment, the determination method for adaptive radiotherapy may involve multiple rounds of radiotherapy. Therefore, each round of the radiotherapy corresponds to a radiotherapy fraction. The current radiotherapy fraction refers to the next radiotherapy fraction to be performed, i.e., the present radiotherapy fraction. The current fraction image refers to a fraction scan image corresponding to the current radiotherapy fraction obtained by scanning the target subject with a scanning device before performing the treatment of the current radiotherapy fraction on the target subject. For example, during the second treatment (the second radiotherapy fraction), a scan image of a potentially diseased part of the patient is collected, and this scan image is used as the fraction scan image for the second radiotherapy fraction.

In an embodiment, when scanning the target subject, imaging methods, such as Computed Tomography (CT), Magnetic Resonance Imaging (MRI), Electronic Portal Imaging Device (EPID), etc., can be used to scan the target subject, thereby obtaining initial images or current fraction images of the target subject.

In an embodiment, the initial image used in a previous radiotherapy plan can be the current fraction image used in one of the historical radiotherapy fractions. The historical radiotherapy fractions may include radiotherapy fractions. In yet another embodiment, the initial image used in the previous radiotherapy plan may be a planning image used for formulating an original radiotherapy plan.

In an embodiment, since the medical process or the condition of the target subject may change, the radiotherapy plan corresponding to the current radiotherapy fraction may be the same as or different from the previous radiotherapy plan. For example, based on an initial radiotherapy plan involving multiple radiotherapy fractions, when the therapeutic effect of the radiotherapy on the target subject meets expectations, radiotherapy may be performed on the target subject in accordance with the number of radiotherapy fractions and the radiotherapy dose specified in the initial radiotherapy plan. When the therapeutic effect of the radiotherapy on the target subject exceeds or fails to meet the expectations set in the radiotherapy plan, the treatment regimen may be revised on the basis of the initial radiotherapy plan to meet the medical requirements of the target subject.

In an embodiment, a new radiotherapy plan for the target subject can also be redefined according to medical requirements. When radiotherapy is performed in the current radiotherapy fraction in accordance with the new radiotherapy plan, the current fraction image of the last radiotherapy fraction in the previous radiotherapy plan (e.g., the initial radiotherapy plan) can be obtained as the initial image.

In an embodiment, when it is determined to perform radiotherapy on the target subject, an initial radiotherapy plan for the target subject can be formulated. Then, the target subject can be scanned to obtain a scan image of the target subject. The scan image can be referred to as the initial image. Based on this initial image, an initial radiotherapy plan for the target subject can be formulated. The content of the initial radiotherapy plan includes the rounds of radiotherapy, radiotherapy parameters for each round of radiotherapy, such as machine parameters, dose parameters, etc. The radiotherapy can include, but is not limited to, the radiotherapy itself, radiotherapy simulation (e.g., using phantoms for verification, simulating the radiotherapy, etc.), or radiation processing.

In an embodiment, for each radiotherapy fraction, before performing the treatment of the current radiotherapy fraction, the current fraction image of the target subject is collected, and the initial image of the target subject is also obtained, to determine whether to trigger adaptive radiotherapy in the current radiotherapy fraction based on a difference between the current fraction image and the initial image. When the difference between the current fraction image and the initial image is small, there is no need to trigger the adaptive radiotherapy, and image-guided radiotherapy can be performed based on a pre-established radiotherapy plan. When the difference between the current fraction image and the initial image is large, the adaptive radiotherapy needs to be triggered, and radiotherapy is performed on the target subject based on an adjusted radiotherapy plan. Since it is not necessary to determine adaptive radiotherapy triggering in each round of radiotherapy, resources can be saved, efficiency can be improved, and the user's workload can be reduced, thereby increasing the number of patients treated or plans verified per radiotherapy device per day.

In an embodiment, when obtaining the initial image of the target subject, the computer device can obtain the initial image from local storage. For example, when the computer device has already obtained the initial image of the target subject during the adaptive trigger determination of the previous radiotherapy fraction, the computer device can save the initial image of the target subject locally this round, so that the computer device can quickly obtain the initial image of the target subject from local storage next time, thereby improving data acquisition efficiency and further improving the trigger efficiency of the adaptive radiotherapy. In addition, the computer device can also obtain the initial image from a preset database according to an identifier of the target subject. The preset database may include, but is not limited to, a Picture Archiving and Communication System (PACS) server.

In Step 204, a trigger analysis result is determined according to the current fraction image, the initial image, and a trigger analysis model.

In Step 206, whether to perform the adaptive radiotherapy is determined based on the trigger analysis result.

In an embodiment, the trigger analysis result can include at least one of a trigger attention parameter and adaptive radiotherapy trigger determination information. The trigger attention parameter can include benefit information of one or more dose indicators.

In an embodiment, the trigger analysis model can output a trigger attention parameter related to adaptive radiotherapy triggering, i.e., output benefit information of one or more dose indicators. The benefit information of a dose indicator can be used to characterize the degree of dosimetric benefit brought to the dose indicator by performing an adaptive radiotherapy adjustment in the current radiotherapy fraction for the target subject, compared to the image-guided radiotherapy plan.

In an embodiment, the benefit information can include the degree or amount of benefit of the dose indicator brought by performing adaptive radiotherapy in the current fraction. For example, compared with conventional Image-Guided Radiotherapy (IGRT), when the adaptive radiotherapy is performed in the current fraction, an irradiated target volume can receive a greater radiation dose (e.g., greater than 30%), or the normal tissues or organs around the target that are not intended to be irradiated can receive a smaller radiation dose (e.g., less than 50%), etc.

In an embodiment, the trigger analysis model can also output the adaptive radiotherapy trigger determination information. The adaptive radiotherapy trigger determination information can indicate whether to trigger the adaptive radiotherapy or not.

In an embodiment, the adaptive radiotherapy trigger determination information output by the trigger analysis model can be determined based on the trigger attention parameter. For example, the trigger attention parameter can be used as an input to the trigger analysis model or a sub-model thereof, or as an intermediate parameter of the trigger analysis model or a sub-model thereof.

In an embodiment, the trigger attention parameter can be an intermediate parameter obtained during the processing of the trigger analysis model when outputting the adaptive radiotherapy trigger determination information, that is, after analyzing and processing the current fraction image and the initial image, the trigger analysis model can obtain the benefit information of one or more dose indicators. Then, the adaptive radiotherapy trigger determination information is determined according to the benefit information of one or more dose indicators.

In an embodiment, the trigger analysis model can also directly output the adaptive radiotherapy trigger determination information based on the current fraction image or the initial image. For example, the trigger analysis model can only output the adaptive radiotherapy trigger determination information, which can be used to prompt the user that the target subject is more suitable for adaptive radiotherapy in the current radiotherapy fraction, and the effect is better compared with image-guided radiotherapy.

In an embodiment, the trigger analysis model can be trained based on historical images and historical adaptive radiotherapy trigger determination results.

In an embodiment, the trigger analysis model can be a machine learning model. The machine learning model includes a neural network model, a decision tree model, etc.

In an embodiment, the trigger attention parameter and the adaptive radiotherapy trigger determination information can also be obtained by the trigger analysis model performing different analyses on the current fraction image and the initial image. For example, the trigger analysis model can obtain the trigger determination information by performing a first analysis on the current fraction image and the initial image, and can obtain the adaptive radiotherapy trigger attention parameter by performing a second analysis on the current fraction image and the initial image.

In an embodiment, the adaptive radiotherapy trigger determination information can include whether to trigger adaptive radiotherapy in the current radiotherapy fraction, and can also include whether to trigger adaptive radiotherapy in the next fraction or subsequent multiple fractions after the current radiotherapy fraction. For example, the adaptive radiotherapy may not be triggered in the current radiotherapy fraction, but which fraction in the subsequent fractions needs to trigger adaptive radiotherapy, or whether all subsequent fractions in the entire treatment do not need to trigger adaptive radiotherapy, etc.

In the above determination method for adaptive radiotherapy, the computer device obtains the current fraction image corresponding to the current radiotherapy fraction of the target subject, and obtains the initial image used when determining the initial radiotherapy plan for the target subject. Then, the trigger analysis result is determined according to the current fraction image, the initial image, and the trigger analysis model, and whether to perform adaptive radiotherapy is determined based on the trigger analysis result. In other words, by using the determination method for adaptive radiotherapy provided in the embodiments of the present disclosure, automatic trigger determination for the adaptive radiotherapy can be achieved through a machine learning model. Compared with the method of manual determination analysis based on experience, the determination method for adaptive radiotherapy not only improves the efficiency of trigger determination for adaptive radiotherapy but also improves the consistency of the trigger determination, avoiding different trigger determination results caused by different users based on different clinical experiences, thereby improving the accuracy of adaptive trigger determination and the rationality of adaptive triggering.

In addition, when the trigger analysis result includes at least one of a trigger attention parameter and adaptive radiotherapy trigger determination information and the trigger attention parameter includes benefit information of one or more dose indicators, using this method can not only provide the user with a more rational and valuable trigger attention parameter but also provide the adaptive radiotherapy trigger determination information, i.e., whether to trigger adaptive radiotherapy, that is, the trigger analysis result obtained by this method can provide the user with reference basis for trigger determination and result verification, and can also provide specific reference information for adaptive radiotherapy adjustment in the current fraction or subsequent fractions, comprehensively improving the accuracy of adaptive trigger determination, and thereby improving the rationality and interpretability of adaptive triggering.

In an embodiment, the adaptive radiotherapy trigger determination information can be obtained through the above trigger analysis model. As shown in FIG. 3, an obtainment method for adaptive radiotherapy trigger determination information is provided. The above trigger analysis model can include a first trigger analysis model. The first trigger analysis model is configured to output the adaptive radiotherapy trigger determination information. As such, Step 204 can include Step 302 to Step 306.

In Step 302, the current fraction image and the initial image are registered to obtain a registered image.

In an embodiment, when performing image registration, rigid registration can be performed on the current fraction image and the initial image. Since the target subject may have differences in position, posture, etc., at different radiotherapy fractions, to ensure that the positions or structures of tissues in two images remain consistent, the current fraction image can be rigidly registered with the initial image, including but not limited to registration operations, such as rotation or translation, that is, the current fraction image can be rotated, translated, etc., so that the adjusted registered image is substantially consistent with the initial image.

In Step 304, a region of interest in the registered image is delineated to obtain a delineated image. The delineated image includes a delineation result of the region of interest.

In an embodiment, the registered image is an image obtained after processing the current scan image through operations such as rotation or translation.

In an embodiment, for the registered image, the region of interest in the registered image can be delineated to obtain a delineated image containing the delineation result of the region of interest. The region of interest can contain at least one of a target volume and an organ at risk around the target volume. The target volume is a lesion area, i.e., an area where rays are concentrated, that needs radiotherapy. The organ at risk around the target volume is a healthy organ that may be irradiated during radiotherapy. During radiotherapy, irradiation to the organ at risk around the target volume, i.e., a healthy organ other than the lesion, should be minimized to reduce the adverse effects of radiotherapy on healthy organs.

In an embodiment, an area within a certain range around the target volume can be taken as the region of interest. The region of interest can contain, but is not limited to, specific parts, organs, tissues, etc., that the user focuses on.

In an embodiment, when delineating the region of interest, manual delineation by the user can be supported, or automatic delineation can be supported. During the automatic delineation, an automatic segmentation model can be used to segment the region of interest in the registered image, and then the region of interest in the registered image is delineated based on a segmentation result to obtain a delineated image containing the delineation result of the region of interest.

In an embodiment, the region of interest in the registered image can also be delineated through deformation registration based on an original delineation result in an original scan image, thereby obtaining a delineated image containing the delineation result of the region of interest. When formulating the initial radiotherapy plan, the user delineates the region of interest in the initial image, i.e., the initial image can include the original delineation result corresponding to the region of interest. For example, the computer device can use a deformation registration algorithm to perform the deformation registration on the initial image and the registered image, thereby obtaining the deformation information between the initial image and the registered image, such as a deformation field. Then, based on this deformation information, the original delineation result in the initial image is registered into the registered image, thereby obtaining the delineated image containing the delineation result of the region of interest.

It should be noted that the method for delineating the region of interest is not limited to the methods described above. The delineation method can be one or a combination of the above delineation methods, or can be other delineation methods or a combination of other delineation methods with the above delineation methods, etc.

In Step 306, the delineated image is input into the first trigger analysis model to output the trigger analysis result.

In an embodiment, the trigger analysis result can include the adaptive radiotherapy trigger determination information.

In an embodiment, the first trigger analysis model can determine the adaptive radiotherapy trigger determination information based on an intermediate parameter, such as the trigger attention parameter, obtained during an analysis. The first trigger analysis model can also determine the adaptive radiotherapy trigger determination information based on other intermediate parameters, or the adaptive radiotherapy trigger determination information can be obtained based on feature information of the delineated image obtained during such as the processing of the first trigger analysis model.

In an embodiment, the registered image obtained in Step 302 can be an adjusted current fraction image or a fused image of the adjusted current fraction image and the initial image. Correspondingly, the delineated image obtained in Step 304 can be a delineated image, including the current delineation result of the region of interest, obtained by delineating the region of interest on the adjusted current fraction image, a delineated image, including the current delineation result of the region of interest, obtained by delineating the region of interest of the fused image, or the original delineation result.

In an embodiment, when the delineated image is a delineated image including the current delineation result, the original delineation result, the current fraction image, and the initial image, the computer device can input this delineated image into the first trigger analysis model for analysis, and obtain the adaptive radiotherapy trigger determination information based on at least one of a feature difference between the current delineation result and the original delineation result and a feature difference between the current fraction image and the initial image. After the analysis by the first trigger analysis model, the trigger analysis result of triggering or not triggering the adaptive radiotherapy can be output.

In an embodiment, when the delineated image is a delineated image including the current delineation result and the current fraction image, the computer device can input this delineated image and the initial image including the original delineation result into the first trigger analysis model for analysis, and then output the trigger analysis result of triggering or not triggering the adaptive radiotherapy based on at least one of the feature difference between the current delineation result and the original delineation result and the feature difference between the current fraction image and the initial image.

In an embodiment, the first trigger analysis model can be any type of machine learning model, including but not limited to a random forest, a decision tree, a support vector machine, a regression model, a deep learning model, etc. The output of the first trigger analysis model can include two approaches, which are regression and classification. When the regression is used, a probability of whether to trigger the adaptive radiotherapy can be output, and by comparing the output probability with a preset probability threshold, a trigger analysis result in the form of classification can be obtained, i.e., triggering or not triggering the adaptive radiotherapy.

In an embodiment, when the first trigger analysis model is a deep learning model, the delineated image or the delineated image and the initial image including the original delineation result, can be input into the deep learning model for feature learning and trigger determination, and finally, the adaptive radiotherapy trigger determination information is output. Using the approach of deep learning can automatically find and learn features, and complete trigger determination, improving the processing efficiency of the first trigger analysis model.

In this embodiment, the trigger analysis model can include a first trigger analysis model. The first trigger analysis model can be used to output a trigger analysis result of whether to trigger the adaptive radiotherapy, i.e., adaptive radiotherapy trigger determination information. When using the first trigger analysis model for trigger determination, the computer device can first register the current fraction image and the initial image to obtain the registered image. Then, the region of interest in the registered image is delineated to obtain the delineated image containing the delineation result of the region of interest. Finally, the delineated image is input into the first trigger analysis model to output the trigger analysis result of whether to trigger the adaptive radiotherapy. That is, in this embodiment, a machine learning algorithm is used to automatically determine whether to trigger the online adaptive radiotherapy and obtain the adaptive radiotherapy trigger determination information, which can save the user the process of repeated confirmation from the treatment on-site, thereby shortening the trigger time of the adaptive plan, and improving trigger efficiency and the consistency of trigger determination results.

In an embodiment, the above first trigger analysis model can be a deep learning model that automatically performs feature learning and trigger determination. In other embodiments, the first trigger analysis model can also be other machine learning models besides deep learning. In this case, feature extraction needs to be performed separately, and the user needs to set the required features.

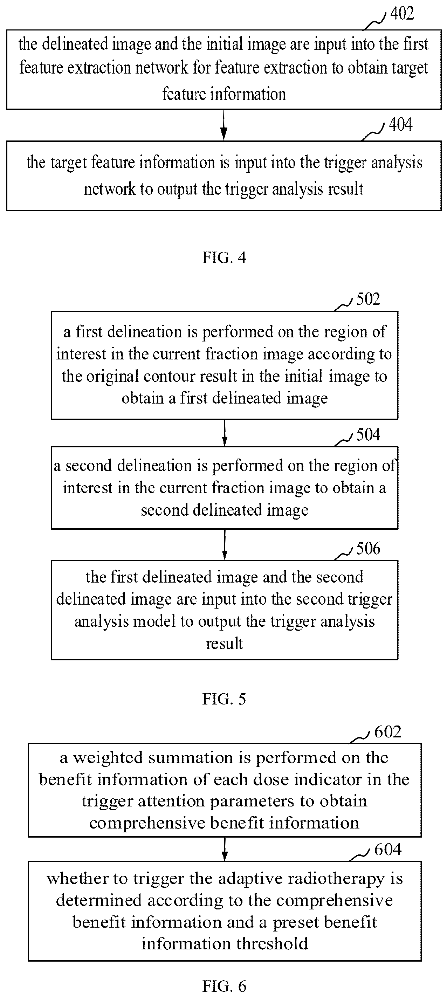

In an embodiment, the first trigger analysis model can include a first feature extraction network and a trigger analysis network. Accordingly, as shown in FIG. 4, above Step 306 can include Step 402 to Step 404.

In Step 402, the delineated image and the initial image are input into the first feature extraction network for feature extraction to obtain target feature information.

In an embodiment, the target feature information can include, but is not limited to, at least one of morphological difference feature information, image difference feature information, or registration difference feature information, based on the region of interest. The initial image includes an original delineation result of the region of interest.

In an embodiment, the delineated image can be an image obtained by delineating the region of interest on the adjusted current fraction image. In this case, the delineated image and the initial image can be input into the first feature extraction network for feature extraction to obtain target feature information.

In an embodiment, the computer device can input the delineated image into the first feature extraction network for morphological feature extraction of the region of interest to obtain morphological feature information of the current delineation result. The computer device inputs the initial image into the first feature extraction network for the morphological feature extraction of the region of interest to obtain morphological feature information of the original delineation result. Then, the computer device can compare the morphological feature information of the current delineation result and the morphological feature information of the original delineation result to obtain the morphological difference feature information of the region of interest. Optionally, the morphological feature information can include, but is not limited to, information such as the position, shape, size, etc., of the region of interest. Correspondingly, the morphological difference feature information can include feature information such as position difference, shape difference, and size difference between the current delineation result and the original delineation result.

In an embodiment, the computer device can also input the delineated image and the initial image into the first feature extraction network for image difference analysis to obtain image difference feature information. The image difference analysis can include, but is not limited to, overall difference analysis of images, difference analysis of the region of interest in the image, difference analysis of pixels, difference analysis of contrast, etc.

In an embodiment, the computer device can also input the delineated image and the initial image into the first feature extraction network for registration difference analysis to obtain registration difference feature information. The registration difference feature information can include, such as deformation field feature information based on the registration.

In an embodiment, when the morphological difference feature information based on the region of interest, the image difference feature information, and the registration difference feature information are obtained, the morphological difference feature information, the image difference feature information, and the registration difference feature information can be merged to obtain the above target feature information.

It should be noted that, the feature extraction and the types of feature information can also be defined by the user, and the user can also manually perform the feature extraction to input the extracted feature information into the trigger analysis network for analysis.

In Step 404, the target feature information is input into the trigger analysis network to output the trigger analysis result.

In an embodiment, the trigger analysis network can be a machine learning network obtained based on one or more modeling methods, such as random forest, decision tree, support vector machine, regression, etc. After the target feature information is obtained through the first feature extraction network, the target feature information can be input into the trigger analysis network for processing to obtain a trigger analysis result of whether to trigger adaptive radiotherapy, i.e., the adaptive radiotherapy trigger determination information.

In this embodiment, the first trigger analysis model can include the first feature extraction network and a machine-learning-based trigger analysis network. The computer device inputs the delineated image and the initial image into the first feature extraction network for feature extraction to obtain target feature information. Then, the target feature information is input into the trigger analysis network to output the trigger analysis result. The initial image includes the original delineation result of the region of interest. By means of the approach in this embodiment, the automatic trigger determination for the adaptive radiotherapy is achieved through a machine learning algorithm, and a direct result of whether to trigger the adaptive radiotherapy is obtained, thereby improving the efficiency of trigger determination and the consistency of trigger determination results.

In an embodiment, by means of the approaches in the above respective embodiments, the adaptive radiotherapy triggering can be achieved. The adaptive radiotherapy triggering is a technology that determines whether or when the adaptive radiotherapy adjustment is needed during the treatment based on patient images, dose information, etc., which can assist the user in determining and thereby improving the treatment efficiency with machines. However, when aiding in determination, the above adaptive radiotherapy triggering can only provide the user with a direct outcome of whether to trigger the adaptive radiotherapy, and thus, the value and rationality for clinical application are low. Compared with providing information on whether to trigger the adaptive radiotherapy, when benefit information on how much adaptive radiotherapy adjustment can bring to the concerned dose indicators, which are compared with image-guided radiotherapy, can be provided, it would be more rational and interpretable in clinical applications. Therefore, using only a model that predicts whether the adaptive radiotherapy needs to be triggered to meet clinical needs is imperfect in clinical practice, which makes the user lack specific reference information when determining whether the adaptive radiotherapy adjustment is needed.

Based on this, another trigger analysis model that can predict the benefit of the adaptive radiotherapy is provided in an embodiment of the present disclosure. The trigger analysis model can predict different dose indicators of clinical concern, thereby assisting clinical evaluation of whether the adaptive radiotherapy is needed for the target subject. By predicting the benefit of different dose indicators, the user can more comprehensively evaluate whether the adaptive radiotherapy adjustment is needed, thereby better balancing the relationship between clinical resources and patient benefits.

In an embodiment, the above trigger analysis model can also include a second trigger analysis model. The second trigger analysis model is configured to output trigger attention information including benefit information of one or more dose indicators. Accordingly, as shown in FIG. 5, above Step 204 can also include Step 502 to Step 506.

In Step 502, a first delineation is performed on the region of interest in the current fraction image according to the original delineation result in the initial image to obtain a first delineated image.

In an embodiment, the initial image includes an original delineation result of the region of interest. For example, when delineating the region of interest in the current fraction image based on the original delineation result, the original delineation result in the initial image can be rigidly copied to the current fraction image to obtain a first delineated image containing the original delineation result.

In an embodiment, the first delineated image can be the current fraction image in combination with the original delineation result, i.e., adding an original delineated outline or original area of the region of interest to the current fraction image. The first delineated image can also be a single image obtained by registration and fusion of the current fraction image and the original delineation result.

In an embodiment, before delineating, rigid registration can be performed on the initial image and the current fraction image in the first place. Then, based on the result of the rigid registration, the original delineation result in the initial image is rigidly copied to the current fraction image to obtain the first delineated image containing the original delineation result. By way of this, the original delineation result of the region of interest in the initial image can be copied to the corresponding position of the region of interest in the current fraction image, avoiding a large position offset between the original delineation result and the region of interest, thereby improving the accuracy of the first delineated image.

It should be noted that, the position and morphology of the region of interest in the current fraction image may be different from those in the initial image, but after rigid copying, it is at least necessary to ensure that the original delineation result corresponds to the region of interest in the current fraction image, rather than other areas outside the region of interest. For example, due to differences in the positioning and posture of the target subject, the region of interest in the initial image is at the center of the entire image, while the region of interest in the current fraction image is at the lower left of the entire image. Then, when the original delineation result is rigidly copied to the current fraction image, it is expected that the original delineation result is located at the position of the region of interest in the lower left, not at the center of the current fraction image.

In Step 504, a second delineation is performed on the region of interest in the current fraction image to obtain a second delineated image.

In an embodiment, the computer device can automatically segment the region of interest in the current fraction image by a preset segmentation algorithm, and then perform the second delineation on the region of interest in the current fraction image based on a segmentation result to obtain the second delineated image including the current delineation result of the region of interest. It should be noted that, the preset segmentation algorithm can be any type of segmentation network or model. The type of the preset segmentation algorithm is not specifically limited in the embodiments of the present disclosure.

In Step 506, the first delineated image and the second delineated image are input into the second trigger analysis model to output the trigger analysis result.

In an embodiment, the first delineated image is the current fraction image containing the original delineation result of the region of interest. The second delineated image is the current fraction image containing the current delineation result of the region of interest. For example, the second trigger analysis model can determine the benefit information of multiple dose indicators by performing difference analysis on the original delineation result of the region of interest and the current delineation result of the region of interest.

In an embodiment, the computer device can input the first delineated image and the second delineated image into the second trigger analysis model for difference analysis to obtain a trigger analysis result including trigger attention parameters.

In an embodiment, the second trigger analysis model can include a second feature extraction network and a decision network. The computer device can input the first delineated image into the second feature extraction network for feature extraction to obtain first feature information, and input the second delineated image into the second feature extraction network for feature extraction to obtain second feature information. Then, the first feature information and the second feature information are input into the decision network to output the trigger analysis result.

In an embodiment, after the first feature information and the second feature information are obtained, the first feature information and the second feature information can be concatenated, and the concatenated feature information is input into the decision network to output the trigger analysis result. It should be noted that, the network structures of the second feature extraction network and the decision network are not specifically limited in the embodiments of the present disclosure. The first feature information and the second feature information can be a first feature vector and a second feature vector, or a first feature map and a second feature map, respectively. The form of the feature information is not specifically limited in the embodiments of the present disclosure.

In an embodiment, for the benefit information of one or more dose indicators in the trigger analysis result, the greater the benefit information, the higher the dosimetric benefit brought by performing adaptive plan adjustment in this radiotherapy fraction for the target subject.

In an embodiment, the benefit information can be, such as a benefit degree or a benefit value.

In this embodiment, the trigger analysis model includes the second trigger analysis model. The second trigger analysis model can be configured to output the trigger attention parameters, i.e., the benefit information of one or more dose indicators. When using the second trigger analysis model for the trigger determination, the computer device performs a first delineation on the region of interest in the current fraction image according to the original delineation result in the initial image to obtain a first delineated image, and performs a second delineation on the region of interest in the current fraction image to obtain a second delineated image. Then, the first delineated image and the second delineated image are input into the second trigger analysis model to output a trigger analysis result including the trigger attention parameters. That is, in this embodiment, in the current radiotherapy fraction, compared with the image-guided radiotherapy, the benefits of the dose indicators brought by the adaptive radiotherapy adjustment can be predicted based on changes in patient anatomy, as an auxiliary decision-making tool to help the user determine whether the adaptive radiotherapy adjustment is needed. This determination method for adaptive radiotherapy can provide more valuable and referential trigger attention information for clinical practice, thus improving the rationality and interpretability of adaptive radiotherapy trigger determination.

In an embodiment, based on the above embodiments, after obtaining the trigger attention information including the benefit information of one or more dose indicators, whether to trigger the adaptive radiotherapy can be further determined to obtain the trigger analysis result of whether to trigger the adaptive radiotherapy based on the trigger attention information.

In an embodiment, when the trigger attention information includes benefit information of one dose indicator, whether to trigger adaptive radiotherapy can be determined based on the relationship between the benefit information of this dose indicator and a preset benefit information threshold. If the benefit information of the dose indicator is greater than or equal to the preset benefit information threshold, a trigger analysis result of triggering adaptive radiotherapy is obtained.

In an embodiment, when the trigger attention information includes the benefit information of multiple dose indicators, whether to trigger adaptive radiotherapy can be comprehensively determined based on the benefit information of the multiple dose indicators. Accordingly, as shown in FIG. 6, the above determination method for adaptive radiotherapy can further include Step 602 to Step 604.

In Step 602, a weighted summation is performed on the benefit information of each dose indicator in the trigger attention parameters to obtain comprehensive benefit information.

In an embodiment, respective weights of the dose indicators can be the same or different. The respective weights of the dose indicators can be flexibly set according to the actual importance of the dose indicators, and then a weighted summation is performed based on the benefit information of the respective dose indicators and the weights of the respective dose indicators to obtain comprehensive benefit information.

In Step 604, whether to trigger the adaptive radiotherapy is determined according to the comprehensive benefit information and a preset benefit information threshold.

In an embodiment, when the comprehensive benefit information is greater than or equal to the preset benefit information threshold, it is determined to trigger the adaptive radiotherapy. When the comprehensive benefit information is less than the preset benefit information threshold, it is determined not to trigger the adaptive radiotherapy.

In this embodiment, after the benefit information of multiple dose indicators is evaluated through the second trigger analysis model, the weighted summation can be performed on the benefit information of each dose indicator in the trigger attention parameters to further obtain the comprehensive benefit information. Then, whether to trigger the adaptive radiotherapy is determined according to the comprehensive benefit information and the preset benefit information threshold. In other words, by means of the determination method for adaptive radiotherapy provided in the embodiments of the present disclosure, not only can the trigger attention parameters of the adaptive radiotherapy be obtained, providing reference information for triggering the adaptive radiotherapy, but also the trigger analysis result of whether to trigger the adaptive radiotherapy, i.e., the adaptive radiotherapy trigger determination information, can be automatically determined based on the trigger attention parameters, providing a direct trigger analysis result to the user. Using the determination method for adaptive radiotherapy can improve the efficiency of the trigger determination and improve the rationality and interpretability of the trigger determination.

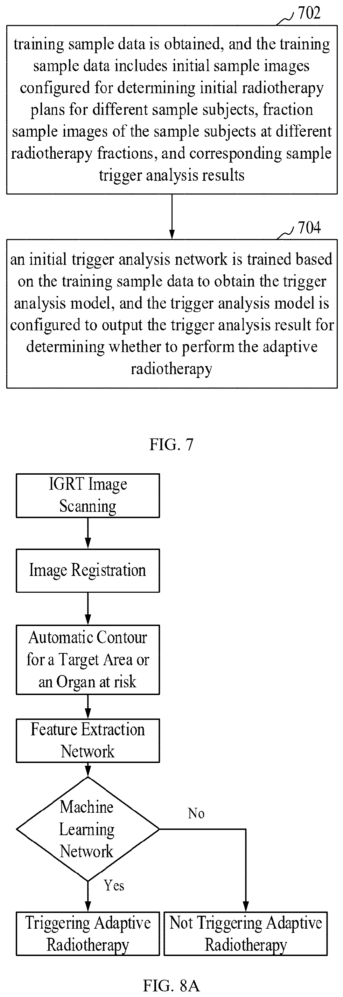

In an embodiment, as shown in FIG. 7, a training method for a trigger analysis model is also provided. The trigger analysis model can be applied to the determination for adaptive radiotherapy. Taking the training method for the trigger analysis model applied to the computer device in FIG. 1 as an example, the training method for the trigger analysis model includes the following Step 702 to Step 704.

In Step 702, training sample data is obtained. The training sample data includes initial sample images used when determining initial radiotherapy plans for different sample subjects, fraction sample images of the sample subjects at different radiotherapy fractions, and sample trigger analysis results corresponding to the different radiotherapy fractions.

In an embodiment, the sample trigger analysis result can include at least one of sample trigger attention parameters and sample adaptive radiotherapy trigger determination information.

In an embodiment, when the sample trigger analysis result includes the sample trigger attention parameters, the sample trigger attention parameters can include benefit information labels for one or more dose indicators.

In an embodiment, obtaining the benefit information labels corresponding to respective fraction sample images can include:
for each fraction sample image, obtaining indicator differences of respective dose indicators obtained after performing an image-guided radiotherapy plan and an adaptive plan at the corresponding radiotherapy fraction of the fraction sample image, respectively; performing normalization on the indicator differences of the respective dose indicators to obtain benefit information labels for the respective dose indicators corresponding to the fraction sample image.

In an embodiment, taking a single dose indicator as an example, when performing the normalization on the indicator difference of the dose indicator, the indicator difference of the dose indicator for each fraction sample image can be normalized based on the indicator differences of the dose indicator for multiple fraction sample images. For example, the maximum indicator difference of the dose indicator can be determined from the indicator differences of the dose indicator for multiple fraction sample images, and the quotient of the indicator difference of the dose indicator for each fraction sample image divided by the maximum indicator difference is taken as the benefit information label of the dose indicator for each fraction sample image. In other words, the indicator differences of the dose indicators for the respective fraction sample images can be normalized to between 0 and 1 to obtain the benefit information labels of the dose indicators. The same normalization can be applied to other dose indicators, which will not be repeated here.

In Step 704, an initial trigger analysis network is trained based on the training sample data to obtain the trigger analysis model. The trigger analysis model is configured to output the trigger analysis result for determining whether to perform the adaptive radiotherapy.

In an embodiment, the initial trigger analysis network can be any type of deep learning network or any type of machine learning network. The type of the trigger analysis network is not specifically limited in the embodiments of the present disclosure.

In an embodiment, the initial sample images and the corresponding fraction sample images in the training sample data can be input into the initial trigger analysis network to obtain an intermediate trigger analysis result. Then, a loss function of the initial trigger analysis network is determined according to the difference between the intermediate trigger analysis result and the corresponding sample trigger analysis result. Further, the network parameters of the initial trigger analysis network are adjusted according to the value of the loss function, and multiple iterations of training are performed until a preset training stop condition is met, and the trigger analysis network that meets the preset training stop condition is taken as a trained trigger analysis model. Optionally, the preset training stop condition can include, but is not limited to, a preset number of iterations, the value of the loss function being less than a preset threshold, etc.

Using the above training method for the trigger analysis model, the trigger analysis model applied to the determination for adaptive radiotherapy can be pretrained. Then, based on the trigger analysis model, comparative analysis between the adaptive radiotherapy and the image-guided radiotherapy can be performed before the performance of the radiotherapy fraction, thereby obtaining a treatment plan with a relatively high benefit degree, which not only improves the efficiency of the trigger determination of the adaptive radiotherapy but also improves the effect and rate of radiotherapy and reduces the rounds of radiotherapy.

In an embodiment, a determination method for triggering based on the first trigger analysis model for determining whether to trigger the adaptive radiotherapy is provided. The first trigger analysis model is configured to obtain the trigger analysis result of whether to trigger the adaptive radiotherapy.

In an embodiment, as shown in FIG. 8A, a first trigger analysis model is shown. The first trigger analysis model includes a feature extraction network (i.e., the above first feature extraction network) and a machine learning model (i.e., the above trigger analysis network). Before the treatment of the current radiotherapy fraction, the first trigger analysis model scans an IGRT image of the target subject as the current fraction image; rigidly registers the IGRT image of the target subject with the initial image of the target subject to obtain the registered image, and delineates the target volume and the organ at risk on the registered image to obtain the delineated image containing the current delineation result; then, uses the feature extraction network to perform feature extraction on the delineated image and the initial image containing the original delineation result to obtain the target feature information; finally, inputs the target feature information into the machine learning model to output the trigger analysis result of whether to trigger the adaptive radiotherapy. The target feature information can include at least one of the morphological difference feature information based on the region of interest, the image difference feature information, or the registration difference feature information.

In an embodiment, the above way of automatically delineating for the target volume and the organ at risk can include, but is not limited to, automatic segmentation models, deformation registration, etc., which can be referred to the description of the related content in the above Step 304 and will not be repeated here. In addition, feature extraction can include multiple forms of features, including but not limited to morphological features based on the Region of Interest (ROI), features based on scanned images, deformation fields based on registration, etc., which can be referred to the description of the related content in the above Step 402 and will not be repeated here. Modelling for the machine learning model can include multiple modeling methods, such as random forest, decision tree, support vector machine, regression, deep learning, etc. The output of the machine learning model can include regression and classification. When the regression is used, a threshold can be designed to convert the regression into a classification, and finally, the trigger analysis result of whether to trigger the adaptive radiotherapy is obtained.

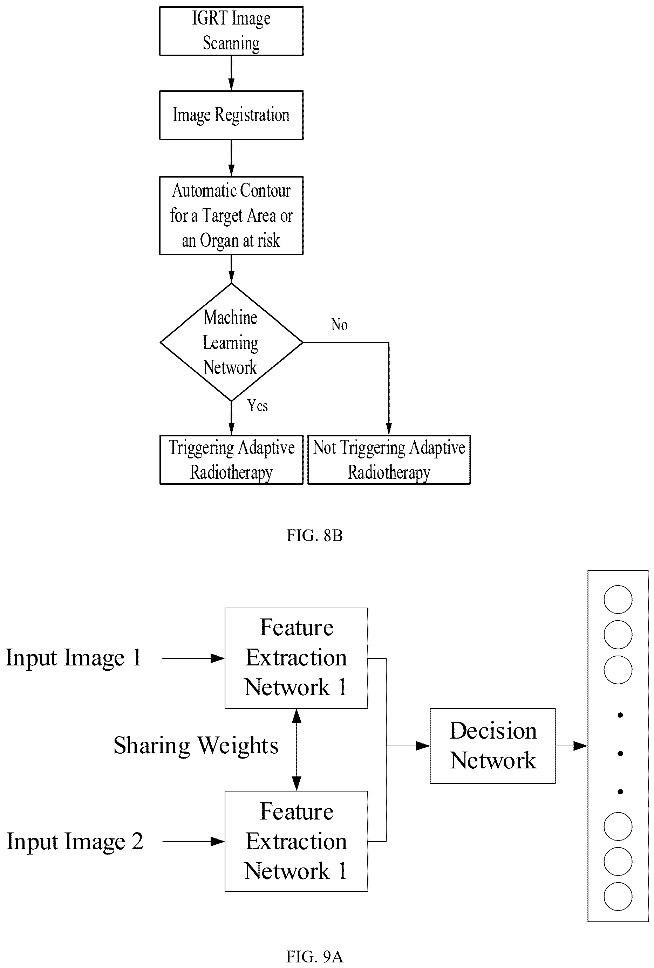

In an embodiment, as shown in FIG. 8B, another first trigger analysis model is shown. The first trigger analysis model can be a deep learning model. Referring to the aforementioned application, before the treatment of the current radiotherapy fraction, the first trigger analysis model scans an IGRT image of the target subject as the current fraction image; rigidly registers the IGRT image of the target subject with an initial image of the target subject to obtain a registered image, and delineates a target volume and an organ at risk on the registered image to obtain a delineated image containing a current delineation result; then, inputs the delineated image and the initial image containing an original delineation result into the deep learning model for feature learning and trigger determination, and outputs the trigger analysis result of whether to trigger the adaptive radiotherapy.

In the above way, whether to trigger the adaptive radiotherapy is automatically determined through a machine learning algorithm, saving the user the repeated confirmation on-site based on the registered image and clinical experience, thereby shortening the trigger determination time for the adaptive radiotherapy, improving the trigger efficiency of the adaptive radiotherapy, and increasing the consistency of trigger determination results.

In an embodiment, as shown in FIG. 9A, a determination method for triggering for determining whether to trigger an adaptive plan based on a second trigger analysis model is provided. The second trigger analysis model can include a feature extraction network (i.e., the above second feature extraction network) and a decision network, where the feature extraction network maps input image information of the second trigger analysis model to a new space, and the decision network makes corresponding determination on each output dose indicator by performing similarity measurement on obtained feature vectors, obtaining the benefit information of each dose indicator.

In an embodiment, the feature extraction network can include feature extraction network 1 and feature extraction network 2. The feature extraction network 1 and feature extraction network 2 can have the same network structure and share parameters, so that the feature extraction network 1 and feature extraction network 2 can obtain more consistent feature map pairs. Each feature extraction network can be composed of 4 convolution blocks, and each convolution block can be composed of a batch normalization layer, a convolution layer, an activation function, and a pooling layer.

Before the treatment of the current radiotherapy fraction, an IGRT image of the target subject is scanned as the current fraction image; the original delineation result of the region of interest of the original scan image is rigidly copied to the current fraction image to obtain a first delineated image as input image 1. The region of interest in the current fraction image is automatically delineated to obtain a second delineated image as input image 2. The first delineated image is input into the feature extraction network 1 for feature extraction to obtain a first feature map. The second delineated image is input into the feature extraction network 2 for feature extraction to obtain a second feature map. The first feature map and the second feature map are then concatenated and sent to the decision network for prediction, outputting multiple nodes. Each node includes the benefit information of a dose indicator of concern. The greater the benefit information of the dose indicator, the higher the dosimetric benefit brought by performing adaptive plan adjustment in the radiotherapy fraction for the target subject.

In an embodiment, the decision network can be composed of two fully connected modules. Each fully connected module includes four parts, which are a batch normalization layer, an activation function layer, a random dropout layer, and a fully connected layer.

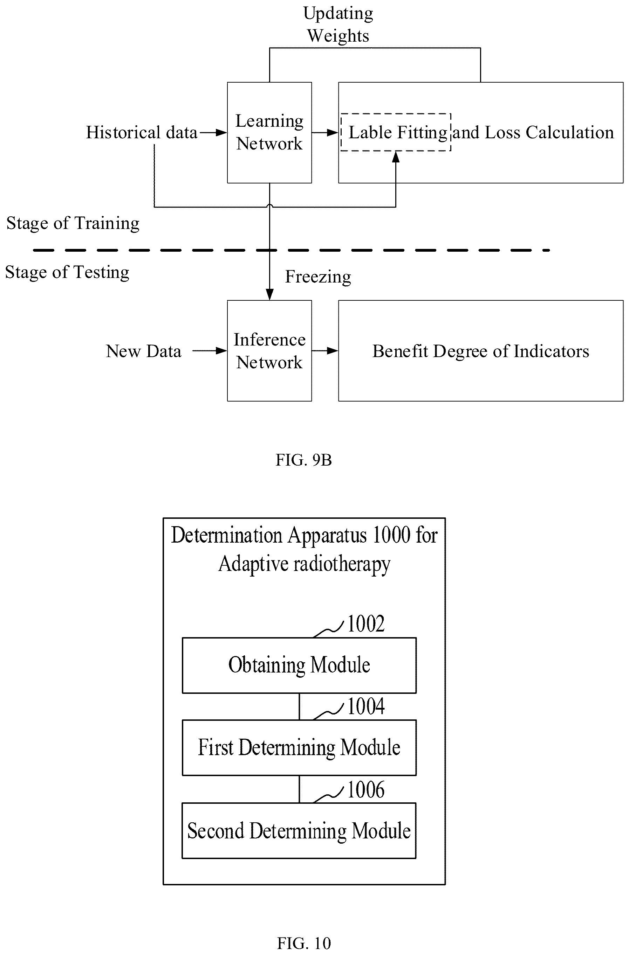

As shown in FIG. 9B, a training of the second trigger analysis model is shown. The second trigger analysis model is required to be trained and tested before use. In the training, label generation is required based on the obtained historical data.

In an embodiment, according to the image-guided radiotherapy plan and the adaptive radiotherapy plan for each radiotherapy fraction, the dose differences of respective dose indicators of clinical concern on the two plans can be obtained, thereby obtaining a set of differences for the respective dose indicators corresponding to fraction sample images of respective radiotherapy fractions. For each dose indicator, by normalizing the set of differences corresponding to the dose indicators, the benefit information label (e.g., a value between 0 and 1) corresponding to the dose indicator for each piece of sample scan data can be obtained. Then, the generated benefit information label can guide the learning network to continuously update weights, and extract and summarize features with the help of the loss function. Depending on the degree of model training, the parameters of the learning network can be selectively frozen for inference in testing.

In the testing, by inputting the scan image of a certain radiotherapy fraction collected into the trained second trigger analysis model for processing, the benefits of the adaptive plan compared with the image-guided radiotherapy plan for each dose indicator of clinical concern can be predicted in real time.

In the above way, a deep-learning-based adaptive radiotherapy trigger analysis model is used. The trigger analysis model can predict the benefits of dose indicators brought by the adaptive plan adjustment compared with the image-guided radiotherapy in the current radiotherapy fraction, based on changes in patient anatomy, as an auxiliary decision-making tool conducive to the user's determination of whether the adaptive radiotherapy adjustment is required. In addition, in this way, the label generation can be automatic, assisting clinicians in providing training labels and reducing clinical workload.

It should be understood that, although the steps in the schematic flowcharts involved in the above embodiments are shown in sequence according to the indication of the arrows in the drawings, these steps are not necessarily executed in sequence according to the orders indicated by the arrows. Unless explicitly stated herein, the execution of these steps is not strictly limited in order, and these steps can be executed in other orders. Moreover, at least a part of the steps in the schematic flowcharts involved in the above embodiments can include multiple steps or multiple stages. These steps or stages are not necessarily executed at the same time, but can be executed at different times. The execution order of these steps or stages is not necessarily sequential but can be executed in turn or alternately with other steps or at least a part of the steps or stages of other steps.

Based on the same inventive concept, a determination apparatus for adaptive radiotherapy for implementing the above determination method for adaptive radiotherapy is further provided in an embodiment of the present disclosure. The implementation for addressing the technical problem provided by the determination apparatus for adaptive radiotherapy is similar to the implementation described in the above determination method for adaptive radiotherapy, so the specific definitions on the determination apparatus for adaptive radiotherapy provided in one or more embodiments below can be referred to the above definitions on the determination method for adaptive radiotherapy, and will not be repeated here.

In an embodiment, as shown in FIG. 10, a determination apparatus 1000 for adaptive radiotherapy is provided, including an obtaining module 1002, a first determining module 1004, and a second determining module 1006.

The obtaining module 1002 is configured to obtain a current fraction image corresponding to a current radiotherapy fraction of a target subject, and obtain an initial image used when determining an initial radiotherapy plan for the target subject.

The first determining module 1004 is configured to determine a trigger analysis result according to the current fraction image, the initial image, and a trigger analysis model.

The second determining module 1006 is configured to determine whether to perform the adaptive radiotherapy based on the trigger analysis result.

In an embodiment, the trigger analysis result includes at least one of trigger attention parameters and adaptive radiotherapy trigger determination information. The trigger attention parameter includes benefit information of one or more dose indicators.

In an embodiment, the trigger analysis model includes a first trigger analysis model. The first determining module 1004 includes:
a registering unit, configured to register the current fraction image and the initial image to obtain a registered image;
a first delineating unit, configured to delineate a region of interest in the registered image to obtain a delineated image, where the delineated image includes a delineation result of the region of interest;
a first determining unit, configured to input the delineated image into the first trigger analysis model to output the trigger analysis result.

In an embodiment, the first trigger analysis model includes a first feature extraction network and a trigger analysis network. The first determining unit is specifically configured to input the delineated image and the initial image into the first feature extraction network for feature extraction to obtain target feature information; and input the target feature information into the trigger analysis network to output the trigger analysis result. The initial image includes an original delineation result of the region of interest.

In an embodiment, the target feature information includes at least one of morphological difference feature information, image difference feature information, or registration difference feature information, based on the region of interest.

In an embodiment, the trigger analysis model includes a second trigger analysis model. The first determining module 1004 includes:
a second delineating unit, configured to perform a first delineation on the region of interest in the current fraction image according to an original delineation result in the initial image to obtain a first delineated image;
a third delineating unit, configured to perform a second delineation on the region of interest in the current fraction image to obtain a second delineated image; and
a second determining unit, configured to input the first delineated image and the second delineated image into the second trigger analysis model to output the trigger analysis result.

In an embodiment, the second delineating unit is specifically configured to perform a rigid copy on the original delineation result from the initial image to the current fraction image to obtain the first delineated image containing the original delineation result.

In an embodiment, the second trigger analysis model includes a second feature extraction network and a decision network. The second determining unit is specifically configured to input the first delineated image into the second feature extraction network for feature extraction to obtain first feature information, input the second delineated image into the second feature extraction network for feature extraction to obtain second feature information, and input the first feature information and the second feature information into the decision network to output the trigger analysis result.

In an embodiment, the trigger analysis result includes trigger attention parameters. The determination apparatus for adaptive radiotherapy further includes:
a processing module, configured to perform a weighted summation on the benefit information of each dose indicator in the trigger attention parameters to obtain comprehensive benefit information; and
a third determining module, configured to determine adaptive radiotherapy trigger determination information according to the comprehensive benefit information and a preset benefit information threshold.

Each module in the above determination apparatus for adaptive radiotherapy can be fully or partially implemented by software, hardware, or a combination thereof. The above modules can be embedded in hardware in or independent of a processor of the computer device, or can be stored in software in a memory of the computer device, so that the processor can call and execute the operations corresponding to the above modules.

Based on the same inventive concept, a training apparatus for a trigger analysis model for implementing the above training method for the trigger analysis model is provided in an embodiment of the present disclosure. The implementation for addressing the technical problem provided by the training apparatus for the trigger analysis model is similar to the implementation described in the above training method for the trigger analysis model. Thus, the specific definitions on the training apparatus for the trigger analysis model provided in one or more embodiments below can refer to the above definitions on the trigger analysis model training method, and will not be repeated here.

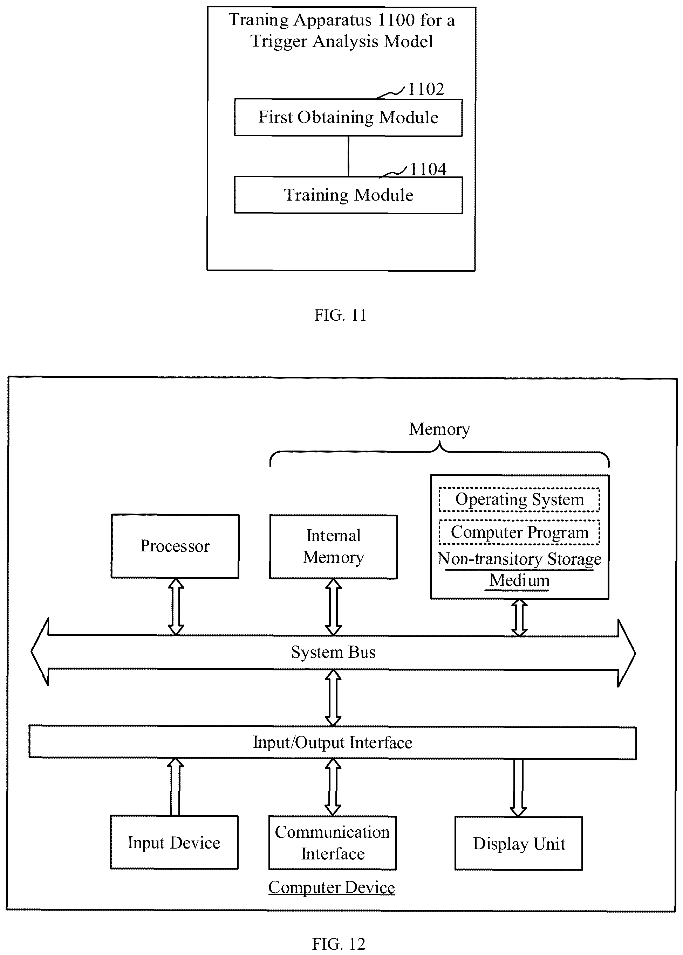

In an embodiment, as shown in FIG. 11, a training apparatus 1100 for a trigger analysis model is provided, including a first obtaining module 1102 and a training module 1104.

The first obtaining module 1102 is configured to obtain training sample data. The training sample data includes initial sample images used when determining initial radiotherapy plans for different sample subjects, fraction sample images of the sample subjects at different radiotherapy fractions, and sample trigger analysis results corresponding to the different radiotherapy fractions.

The training module 1104 is configured to train an initial trigger analysis network based on the training sample data to obtain the trigger analysis model. The trigger analysis model is configured to output at least one of the trigger attention parameters and the adaptive radiotherapy trigger determination information determined based on the trigger attention parameters.

In an embodiment, the sample trigger analysis result includes sample trigger attention parameters. The sample trigger attention parameter includes benefit information labels for one or more dose indicators. The training apparatus for the trigger analysis model further includes:
a second obtaining module, configured to, for each fraction sample image, obtain indicator differences of respective dose indicators obtained after performing an image-guided radiotherapy plan and an adaptive plan at the corresponding radiotherapy fraction of the fraction sample image, respectively; and
a processing module, configured to perform normalization on the indicator differences of the respective dose indicators to obtain benefit information labels for one or more dose indicators corresponding to the fraction sample image.

Each module in the above training apparatus for the trigger analysis model can be fully or partially implemented by software, hardware, or a combination thereof. The above modules can be embedded in hardware in or independent of a processor of the computer device, or can be stored in software in a memory of the computer device, so that the processor can call and execute the operations corresponding to the above modules.

In an embodiment, a computer device is provided. The computer device can be a terminal, a server, or a radiotherapy device. Taking the computer device as a terminal as an example, an internal structure diagram of the computer device can be as shown in FIG. 12. The computer device includes a processor, a memory, an input/output interface, a communication interface, a display unit, and an input device. The processor, the memory, and the input/output interface are connected through a system bus. The communication interface, the display unit, and the input device are connected to the system bus through the input/output interface. The processor of the computer device is configured to provide computing and control capabilities. The memory of the computer device includes a non-transitory storage medium and an internal memory. The non-transitory storage medium stores an operating system and a computer program. The internal memory provides an environment for the operation of the operating system and the computer program in the non-transitory storage medium. The input/output interface of the computer device is configured for information exchange between the processor and an external device. The communication interface of the computer device is configured for wired or wireless communication with an external terminal. The wireless communication can be implemented through Wireless Fidelity (Wi-Fi), a mobile cellular network, Near Field Communication (NFC), or other technologies. The computer program is executed by the processor to implement the determination method for adaptive radiotherapy or the training method for the trigger analysis model. The display unit of the computer device is configured to form visible images. The display unit can be a display screen, a projection device, or a virtual reality imaging device. The display screen may be a liquid crystal display screen or an electronic ink display screen. The input device of the computer device may be a touch layer covered on the display screen, or buttons, a trackball, or a touchpad provided on the housing of the computer device, or can be an external keyboard, a touchpad, or a mouse.

The structure shown in FIG. 12 is only a part of a structure related to the computer device of the present disclosure, and does not constitute a limitation on the computer device to which the technical solution of the present disclosure is applied. A specific computer device may include more or fewer components than shown in the drawings, or combine some components, or have different component arrangements.

In an embodiment, a computer device is provided, including a memory and a processor. The memory stores a computer program. The processor executes the computer program to implement the determination method for adaptive radiotherapy or the training method for the trigger analysis model, according to any one of the above embodiments.

In an embodiment, a non-transitory computer-readable storage medium is provided, having a computer program stored thereon. The computer program is executed by a processor to implement the determination method for adaptive radiotherapy or the trigger analysis model training method, according to any one of the above embodiments.

In an embodiment, a computer program product is provided, including a computer program. The computer program is executed by a processor to implement the determination method for adaptive radiotherapy or the training method for the trigger analysis model, according to any one of the above embodiments.

It should be noted that, the data involved in this disclosure (including but not limited to data used for analysis, stored data, displayed data, etc.) is authorized information or data. The collection, use, or processing, of relevant data, is required to comply with relevant regulations.

Those of ordinary skill in the art can understand that all or part of the processes in the methods of the above embodiments can be implemented by instructing relevant hardware through a computer program. The computer program can be stored in a non-transitory computer-readable storage medium. The computer program, when executed, implements the processes of the embodiments of the above methods. Any reference to the memory, a database, or other media in the embodiments provided in this disclosure can include at least one of a non-transitory or a transitory memory. The non-transitory memory can include a Read-Only Memory (ROM), a magnetic tape, a floppy disk, a flash memory, an optical memory, a high-density embedded non-transitory memory, a Resistive Random-Access Memory (ReRAM), a Magnetoresistive Random Access Memory (MRAM), a Ferroelectric Random Access Memory (FRAM), a Phase Change Memory (PCM), a graphene memory, etc. The transitory memory can include a Random Access Memory (RAM), an external cache memory, etc. As an explanation and not a limitation, the RAM can be in various forms, such as a Static Random Access Memory (SRAM), a Dynamic Random Access Memory (DRAM), etc. The database involved in the embodiments provided in the present disclosure can include at least one of a relational database and a non-relational database. The non-relational database can include such as a blockchain-based distributed database, and the form of the non-relational database is not limited herein. The processor involved in the embodiments provided in the present disclosure can include a general-purpose processor, a central processing unit, a graphics processor, a digital signal processor, a programmable logic device, a data processing logic device based on quantum computing, etc. The type of the processor is not limited herein.

The technical features of the above embodiments can be combined arbitrarily. For the sake of a concise description, not all possible combinations of the technical features in the above embodiments are described. However, as long as there is no contradiction in the combination of these technical features, the combinations should be considered as falling within the scope described in this specification.

The above embodiments only express several implementations of the present disclosure. The description thereof is detailed, but should not be understood as limiting the scope of protection of the present disclosure. It should be pointed out that, for those of ordinary skill in the art, without departing from the inventive concept of the present disclosure, some modifications or improvements can be made to the technical solutions. The modifications or improvements all fall within the scope of protection of the present disclosure. Therefore, the scope of protection of the present disclosure should be subject to the appended claims.

## Claims

1. A determination method for adaptive radiotherapy, comprising:
obtaining a current fraction image corresponding to a current radiotherapy fraction of a target subject, and obtaining an initial image configured for determining a previous radiotherapy plan for the target subject;
determining a trigger analysis result according to the current fraction image, the initial image, and a trigger analysis model; and
determining whether to perform adaptive radiotherapy based on the trigger analysis result.

2. The determination method according to claim 1, wherein the trigger analysis result comprises at least one of trigger attention parameters and adaptive radiotherapy trigger determination information, and the trigger attention parameters comprise benefit information of one or more dose indicators.

3. The determination method according to claim 1, wherein the trigger analysis model comprises a first trigger analysis model, and determining the trigger analysis result according to the current fraction image, the initial image, and the trigger analysis model comprises:
registering the current fraction image with the initial image to obtain a registered image;
delineating a region of interest in the registered image to obtain a delineated image, wherein the delineated image comprises a delineation result of the region of interest; and
inputting the delineated image into the first trigger analysis model to output the trigger analysis result.

4. The determination method according to claim 3, wherein the first trigger analysis model comprises a first feature extraction network and a trigger analysis network, and inputting the delineated image into the first trigger analysis model to output the trigger analysis result comprises:
obtaining target feature information by inputting the delineated image and the initial image into the first feature extraction network for feature extraction; and
outputting the trigger analysis result by inputting the target feature information into the trigger analysis network, wherein the initial image comprises an original delineation result of the region of interest.

5. The determination method according to claim 4, wherein the target feature information comprises at least one of morphological difference feature information, image difference feature information, or registration difference feature information, based on the region of interest.

6. The determination method according to any one of claims 1-5, wherein the trigger analysis model comprises a second trigger analysis model, and determining the trigger analysis result according to the current fraction image, the initial image, and the trigger analysis model comprises:
performing a first delineation on the region of interest in the current fraction image according to an original delineation result in the initial image to obtain a first delineated image;
performing a second delineation on the region of interest in the current fraction image to obtain a second delineated image; and
inputting the first delineated image and the second delineated image into the second trigger analysis model to output the trigger analysis result.

7. The determination method according to claim 6, wherein performing the first delineation on the region of interest in the current fraction image according to the original delineation result in the initial image to obtain the first delineated image comprises:
performing a rigid copy on the original delineation result from the initial image to the current fraction image to obtain the first delineated image containing the original delineation result.

8. The determination method according to claim 6, wherein the second trigger analysis model comprises a second feature extraction network and a decision network, and inputting the first delineated image and the second delineated image into the second trigger analysis model to output the trigger analysis result comprises:
inputting the first delineated image into the second feature extraction network for feature extraction to obtain first feature information;
inputting the second delineated image into the second feature extraction network for feature extraction to obtain second feature information; and
inputting the first feature information and the second feature information into the decision network to output the trigger analysis result.

9. The determination method according to any one of claims 3-5, wherein delineating the region of interest in the registered image comprises delineating the region of interest in the registered image using an automatic segmentation model.

10. The determination method according to any one of claims 3-5, wherein delineating the region of interest in the registered image comprises delineating the region of interest in the registered image through deformation registration based on an original delineation result in the initial image.

11. The determination method according to any one of claims 3-5, wherein the registered image is an adjusted current fraction image, or a fused image of the adjusted current fraction image and the initial image.

12. The determination method according to any one of claims 3-5, wherein the first trigger analysis model is configured to output a probability of whether to trigger the adaptive radiotherapy, and the determination method further comprises comparing the probability with a preset probability threshold to determine whether to trigger the adaptive radiotherapy.

13. The determination method according to claim 4 or 5, wherein obtaining the target feature information comprises obtaining the target feature information by merging the morphological difference feature information, the image difference feature information, and the registration difference feature information.

14. A training method for a trigger analysis model, wherein the trigger analysis model is applied to determination for adaptive radiotherapy, and the training method for the trigger analysis model comprises:
obtaining training sample data, wherein the training sample data comprises initial sample images used when determining a previous radiotherapy plan for different sample subjects, fraction sample images of the sample subjects at different radiotherapy fractions, and sample trigger analysis results corresponding to the different radiotherapy fractions; and
training an initial trigger analysis network based on the training sample data to obtain the trigger analysis model, wherein the trigger analysis model is configured to output a trigger analysis result for determining whether to perform adaptive radiotherapy.

15. A non-transitory computer-readable storage medium, having a computer program stored thereon, wherein the computer program, when executed by a processor, causes the processor to perform the determination method for adaptive radiotherapy according to any one of claims 1-13 or the training method for the trigger analysis model according to claim 14.
